# EUROPÄISCHE PATENTSCHRIFT

(11) **EP 0 282 441 B1**
(45) Veröffentlichungstag und Bekanntmachung des Hinweises auf die Patenterteilung: **16.06.1993**
(21) Anmeldenummer: 88730057.2
(22) Anmeldetag: 09.03.1988
(51) Int. Cl.: G01N 33/18

(54) **Verfahren zur Bestimmung des chemischen Sauerstoffbedarfs von Wasser**
Method for determining the chemical oxygen demand of water
Procédé de détermination de la demande chimique en oxygène de l'eau

(30) Priorität: 09.03.1987 DE 3707815
(43) Veröffentlichungstag der Anmeldung: 14.09.1988
(73) Patentinhaber: Pilz, Ulrich, Dr.-Ing., 13465 Berlin (DE)
(72) Erfinder: Pilz, Ulrich, Dr.-Ing., 13465 Berlin (DE)
(74) Vertreter: Christiansen, Henning, Dipl.-Ing.

(56) Entgegenhaltungen:
- DE-A- 2 135 949
- DE-A- 2 137 073
- DE-A- 2 628 083
- DE-A- 3 217 987
- ANALYTICAL CHEMISTRY, Band 46, Nr. 11, September 1974, Washington, DC (US); G. BELANGER, Seiten 1576-1577#
- PATENT ABSTRACTS OF JAPAN, Band 8, Nr. 51 (P-259)(1488), 08 März 1984#

## Beschreibung

Die Erfindung betrifft ein Verfahren der im Oberbegriff des Anspruchs 1 angegebenen Art sowie eine Verwendung einer Vorrichtung zur Durchführung des Verfahrens.

Die Bestimmung des chemischen Sauerstoffbedarfs (CSB) nimmt bei der Untersuchung von Wasserproben und bei der Bewertung verschiedener Abwasserqualitäten eine hervorragende Rolle ein. Diesem Parameter liegt die Überlegung zugrunde, sämtliche Wasserinhaltsstoffe, deren Umsetzung im biologischen Gewässerreinigungsprozess mit einem Verbrauch von Sauerstoff einhergeht, auf chemischem Wege - und damit wesentlich schneller als in der Natur - zu erfassen.

Der Parameter CSB gehört durch die zentrale Rolle, die gerade der Sauerstoffverbrauch von Gewässerverunreinigungen bei der Wasseruntersuchung spielt, zu den wichtigsten Größen, durch die eine Wasserprobe zu kennzeichnen und zu bewerten ist. Dies kommt auch dadurch zum Ausdruck, daß der CSB in nahezu alle im Bereich der Abwasserkontrolle sowie der Gewässer- und Einleiterüberwachung gültige gesetzliche Bestimmungen (z.B. dem Abwasserabgabengesetz) Eingang gefunden hat.

Gegenwärtig beruhen sämtliche Methoden, den CSB einer Wasserprobe zu bestimmen, auf Verfahren, bei denen die oxidierbaren Wasserinhaltsstoffe bei aggressiven chemischen Bedingungen (hohe Temperatur, stark saures Milieu) mit einem starken Oxidationsmittel (z.B. K₂MnO₄ oder K₂CR₂O₇) in Kontakt gebracht werden. Diese naß-chemischen Methoden haben sich mittlerweile in DIN-Normen niedergeschlagen. Darüberhinaus gibt es zu diesen Methoden zahlreiche Varianten nen (z.B. den Einsatz bestimmter Katalysatoren), welche zum Ziele haben, bestimmten Wasserinhaltsstoffen gerecht zu werden.

Die soeben genannten, in der Laborroutine gut eingeführten Verfahren, sind jedoch aus verschiedenen Gründen nachteilig:
- Sie arbeiten nicht kontinuierlich und sind daher nur zur Untersuchung von Einzelproben geeignet.
- Ihr Einsatz im Rahmen einer zunehmend geforderten kontinuierlichen Gewässer- oder Einleiterüberwachung ist nicht möglich. Statt dessen ist mit großem Aufwand die Untersuchung zahlreicher Einzelproben erforderlich.
- Die Verfahren sind arbeitsaufwendig, brauchen zur Vorbereitung und Durchführung im Labor viel Zeit und verursachen daher hohe Kosten.
- Die Verfahren arbeiten mit gefährlichen Chemikalien; die Analysenrückstände müssen nach entsprechenden Vorschriften aufwendig entsorgt werden.

Weitere Verfahren sind bekannt aus der DE-A1-2 135 949, der US-B1-75 438 und der DE-A1-2 137 073.

Die in der DE-A1-2 135 949 und der US-B1-75 438 beschriebenen Meßverfahren sind in ihrem Funktionsprinzip grundsätzlich gleich. Es wird die Methode der Wasserelektrolyse angewandt, wobei die Wasserstoff entwickelnde Elektrode sich in einem getrennten Raum befindet, der über eine Membran jedoch elektrisch leitend mit der Wasserprobe verbunden ist, in die die Sauerstoff entwickelnde Elektrode eintaucht. Die Messung beruht darauf, daß die durch den Stromfluß theoretisch erzeugte Sauerstoffmenge mit der tatsächtlich erzeugten verglichen wird; erstere wird über die verbrauchte Ladungsmenge bestimmt, während letztere manometrisch als Druckerhöhung gemessen wird.

Bleibt die tatsächtlich gebildete Sauerstoffmenge hinter dem theoretisch erwarteten Wert zurück, so ist der Grund dafür, daß entweder Ladungen für eine direkte Oxidation von Wasserinhaltsstoffen verbraucht wurde, oder der entwickelte Sauerstoff teilweise als Oxidationsmittel verbraucht wurde.

Bei dieser Methode ist das Meßergebnis systemspezifisch mit erheblichen Fehlern behaftet. Auch ist eine kontinuierliche Messung nicht ohne weiteres möglich.

Das in der DE-A1-2 137 073 beschriebene Meßverfahren weicht dagegen von der aus den vorgenannten Schriften bekannten Methoden grundsätzlich ab. Bei der Meßapparatur gemäß DE-A1-2 137 073 handelt es sich um eine rohrartige Meßzelle, durch die die zu untersuchende Probe kontinuierlich gefördert wird, nachdem sie zuvor stark aufgeheizt (40°C) und zudem mit einer Mineralsäure angesäuert wurde.

Zwei an der Rohrwandungen angebrachte Metallelektroden vollziehen nun in der Wasserprobe eine Elektrolyse, d.h. an ihnen entwickelt sich theoretisch Sauerstoff bzw. Wasserstoff. Eine dritte Elektrode durchläuft einen Potentialbereich, der zwischen den elektrochemischen Potentialen der beiden anderen Elektroden liegt; der durch sie fließende Strom wird als Funktion des Elektrodenpotentials aufgezeichnet. Die zuletzt genannte Elektrode nimmt dabei elektrochemische Bedingungen an, die sowohl reduzierend als auch oxidierend wirken; so ist es je nach Potential möglich sowohl gelösten Sauerstoff zu reduzieren als auch Wasserinhaltsstoffe direkt zu oxidieren. Dieses Verfahren ist umständlich und ungenau, so daß ebenfalls eine kontinuierliche Messung nicht möglich ist.

Weiterhin ist aus Analytical Chemistry, Vol. 46, Nr. 11 (1974) Seite 1576-1577 ein Verfahren zur Messung des Schwefeldioxidgehalts der Luft bekannt, wobei mit einer potentiostatischen Meßanordnung ausschließlich eine spezifische anodische Oxidation des Schwefeldioxids zu Sulfat angestrebt wird. Dabei kommt es nicht zur Oxidation von Kohlenwasserstoff- oder anderen Verbindungen.

Der Erfindung liegt die Aufgabe zugrunde, ein Verfahren der eingangs genannten Gattung anzugeben, welches einfacher handhabbar, schnell und zuverlässig sowie vor allem kontinuierlich arbeitet sowie eine Verwendung einer Vorrichtung zur Durchführung des Verfahrens anzugeben.

Diese Aufgabe wird mit den im kennzeichnenden Teil des ersten bzw. letzten Anspruchs angegebenen Maßnahmen gelöst.

Die erfindungsgemäße Meßmethode beruht auf der Erkenntnis, daß zur elektrochemischen Bestimmung des Sauerstoffbedarfs die Bildung von Ozon und OH-Radikalen (nicht zu verwechseln mit OH-Ionen) an einer Bleidioxidelektrode besonders geeignet ist. Ozon und OH-Radikale sind sehr reaktive Oxidationsmittel, die unter bestimmten Voraussetzungen elektrochemisch an Bleioxid entwickelt werden; ihre Bildungsrate - und damit auch ihr durch die Oxidation der Inhaltsstoffe einer Probenlösung verursachter Verbrauch - kann sehr einfach und genau durch die Messung des elektrischen Stromes erfolgen, der durch die Bleidioxidelektrode fließt. Wichtig ist dabei, daß nicht gewöhnlicher Sauerstoff als Oxidationsmittel dient, sondern sehr reaktionsstarke Moleküle.

Um an einer Bleidioxidelektrode definiert und reproduzierbar Ozon und OH-Radikale zu entwickeln, muß sie bei einem definierten elektrochemischen Potential betrieben werden. Hierzu wird eine spezielle Spannungsquelle verwendet, die die Spannung zwischen einer Referenzelektrode und einer Bleidioxidelektrode konstant hält und gleichzeitig den im Stromkreis fließenden Strom über die Bleidioxidelektrode und eine Gegenelektrode (und keinesfalls über die Referenzelektrode) fließen läßt, so daß eine Spannungsquelle nach Art eines Potentiostaten entsteht.

Der Potentiostat selbst und die im erfindungsgemäßen Verfahren verwendete Dreielektrodenanordnung aus Arbeitselektrode (Bleidioxid), Gegenelektrode und Referenzelektrode ist in der elektrochemischen Meßtechnik an sich bekannt. Auch die Tatsache, daß speziell Bleidioxid in wäßriger Lösung elektrochemisch Ozon und OH-Radikale zu erzeugen vermag, ist vom Grundsatz her bekannt. Vollkommen neu ist allerdings, daß Ozon und OH-Radikale an Bleidioxid elektrochemisch erzeugt und zur Messung des CSB eingesetzt werden; neu ist ebenfalls, daß hierzu die Bleidioxidelektrode bei einem konstanten elektrochemischen Potential definiert und reproduzierbar betrieben wird, wobei der durch sie fließende Strom als Maß für den CSB der Wasserprobe dient.

Die Oxidation der organischen Substanz erfolgt bei dem erfindungsgemäßen Verfahren unspezifisch durch OH-Radikale und Ozon, die unter bestimmten Voraussetzungen von einer Bleidioxidelektrode in wäßriger Lösung gebildet werden. OH-Radikale oder Ozon fungieren damit als Oxidanzien.

Die quantitative Freisetzung von Ozon und OH-Radikalen ist eine Eigenschaft, die in der Elektrochemie bislang nur bei dem Elektrodenmaterial Bleidioxid beobachtet wurde. Andere Elektrodenmaterialien (Platin, Nickel, Titan, etc.) sind hierzu nur in wesentlich geringerem Maße in der Lage; an ihnen entwickelt sich im Gegensatz zum Bleidioxid hauptsächtlich Sauerstoff.

An Bleidioxid entwickelt sich demgegenüber erst bei sehr hohen Potentialen Sauerstoff, da bei diesem Elektrodenmaterial die Ozonentwicklung und die Bildung von OH-Radikalen Vorrang haben. Der Effekt, daß Sauerstoff erst bei höheren Potentialen generiert wird als thermodynamisch zu erwarten wäre, wird auch als Sauerstoffüberspannungseffekt bezeichnet; von allen Elektrodenmaterialien hat Bleidioxid die höchste Sauerstoffüberspannung.

Die Wirkung der erfindungsgemäßen Lösung beruht auf der indirekten Oxidation durch Ozon bzw. OH-Radikale, deren Neubildung und Verbrauch durch den über die Arbeitselektrode fließenden Strom bestimmt wird und nicht auf der direkten elektrochemischen Oxidation oder Oxidation durch Sauerstoff, wobei das Elektrodenmaterial praktisch gleichgültig wäre. Demgegenüber wird bei dem erfindungsgemäßen Verfahren die Sauerstoffentwicklung bei möglichst vermieden; sie bildet lediglich einen Störeffekt, der das eigentliche Meßsignal überdeckt. Auch ist einzig und allein Bleidioxid aufgrund seiner hohen Sauerstoffüberspannung und der Tatsache, daß es quantitativ Ozon und OH-Radikale zu erzeugen vermag, als Elektrodenmaterial geeignet. Metallelektroden, wie bei den bekannten Methoden Anwendung finden, wären als Material für die Arbeitselektrode demgegenüber unbrauchbar.

Wesentlich für das erfindungsgemäße Meßverfahren ist, daß erstmalig die Arbeitselektrode auf ein definiertes Potential festgelegt wird. Dies geschieht durch die Verwendung eines Potentiostaten und einer Bezugselektrode mit stabilem Potential, welches beides Bestandsteile des Meßaufbaus sind.

Bei der Bezugselektrode handelt es sich um eine Elektrode 2. Art, die ein definiertes und absolut stabiles Potential aufweist. Eine Elektrode 2. Art besteht aus einem Metallteil (besonders geeignet sind dazu Silber oder Quecksilber), das in eine Lösung eintaucht, in der Ionen desselben Metalls enthalten sind. Diese Anordnung (Lösung und Metallkörper) ist von einem Glasbehältnis umschlossen, das in die Meßlösung eintaucht und mit ihr über ein winziges "Fenster" (Kapillare, poröse Keramik, etc.) in elektrisch leitendem Kontakt steht. Durch diese winzige Verbindung darf keine Meßlösung in den Elektrodeninnenraum gelangen, sondern allenfalls etwas der Elektrodenfüllung in die Probe einfließen (Auflaufraten, die für gute Elektroden typisch sind, liegen bei 1 µl/Tag). Eine solche Elektrode 2. Art hat ein über lange Zeit stabiles Potential, das entsprechend der Gleichung von Nernst berechnet werden kann und das nur beeinträchtigt werden kann, wenn sich ihre Elektrolytzusammensetzung oder ihr metallischer Körper verändern; letzteres kann allerdings nur bei einem falschen Umgang mit der Elektrode auftreten, wenn beispielsweise Meßlösung in den Elektrodenraum vordringt (um dies zu vermeiden, muß der Flüssigkeitsstand im Elektrodenraum immer höher sein als der der Meßlösung) oder ein Strom durch die Elektrode fließt, der elektrolytische Prozesse auslöst (z.B. Wasserstoffentwicklung oder Metallabscheidung).

Im Gegensatz zu einer Elektrode 2. Art besteht eine Elektrode 1. Art aus einem einfachen Metallteil, das direkt in eine Meßlösung getaucht wird. Auch das Verhalten einer solchen Elektrode folgt der Gleichung von Nernst, jedoch ist die Lösung, in die das Metall eintaucht, nicht immer gleich, sondern unterliegt Variationen, die von den Inhaltsstoffen der Meßlösung abhängen. Entsprechend ändert sie auch das Potential einer Elektrode 1. Art sobald Art und Menge der in der Meßlösung vorhandenen Stoffen variieren. Als Bezugssystem vollends unbrauchbar wird eine Elektrode 1. Art, wenn zusätzlich noch ein Strom durch sie fließt; denn dann verändert sie selbst die Inhaltstoffe der Meßlösung und verschiebt dadurch ihr eigenes Potential während des Meßprozesses.

Damit die erfindungsgemäße Meßmethode funktionieren kann, bedarf es neben einer stabilen Bezugselektrode (Elektrode 2. Art) zusätzlich einer Spannungsquelle. Ein typisches Arbeitspotential für die Bleidioxidelektrode liegt z.B. bei 1600 mV. Da das Potential der Bezugselektrode nur 660 mV beträgt (es wird vorzugsweise ein Hg/Hg₂₊-System eingesetzt), muß also die Spannungsquelle eine Potentialdifferenz (Spannung) von 940 mV besitzen. Diese Spannungsquelle muß aber gewährleisten, daß durch die Bezugselektrode kein Strom fließt, da dies wie bereits gesagt zu Veränderungen der Bezugselektrode führen würde. Andererseits muß aber durch die Arbeitselektrode den Strom fließen, der zur Erzeugung des Ozons und der OH-Radikale notwendig ist.

Daher ist die Spannungsquelle so ausgebildet, daß sie den Strom, der durch die Arbeitselektrode fließen muß, durch die Bezugselektrode aber nicht fließen darf, über eine dritte Elektrode in die Meßlösung ableitet. Eine solchermaßen ausgeführte Spannungsquelle nennt man in der Elektrochemie einen Potenstiotasten, während sich für die dritte Elektrode, die "stellvertretend" für die Bezugselektrode den Stromfluß übernimmt, der Begriff "Gegenelektrode" eingebürgert hat.

Die erfindungsgemäße Methode benutzt in diesem Zusammenhang erstmalig einen Potentiostaten und eine stromfrei gehaltene Bezugselektrode. Damit wird erreicht, daß die Arbeitselektrode bei einem definierten Potential elektrochemisch arbeitet, wobei Bleidioxid - wie erwähnt - als Material von entscheidender Bedeutung ist.

Zusammenfassend lassen sich die folgenden wesentlichen Maßnahmen hervorheben:
- Die Nutzung von Ozon und OH-Radikalen als elektrochemisch erzeugte Oxidationsmittel, deren Bildungsrate und Verbrauch über den zu ihrer Erzeugung erforderlichen Stromfluß erfaßt wird.
- Der Einsatz von Bleidioxid als Elektrodenmaterial, das allein in der Lage ist, die zur indirekten Oxidation notwendigen OH- Radikale und das Ozon in wäßriger Lösung quantitativ zu erzeugen.
- Die Verwendung einer stromfrei gehaltenen Bezugselektrode (Elektrode 2.Art), die in Verbindung mit einem Potentiostaten ein konstantes elektrochemisches Potential für die Bleidioxidelektrode als Arbeitselektrode gewährleistet. Die Besonderheit eines Potentiostaten besteht dabei darin, daß er im Gegensatz zu einer normalen Spannungsquelle in der Lage ist, keinen Strom durch die Bezugselektrode fließen zu lassen, sondern hierfür eine dritte Elektrode (die sog. Gegenelektrode) zu nutzen.

Die erfindungsgemäße Meßmethode zur elektrochemischen Erfassung des CSB bildet eine Alternative zu den gebräuchlichen naßchemischen Methoden. Die bei der elektrochemischen Oxidation zu benutzende Meßanordnung ist grundsätzlich bekannt und in den einschlägigen Lehrbüchern der Elektrochemie beschrieben.

Die Funktionsweise der Meßverfahrens beruht darauf, daß an einer sogenannten Arbeitselektrode die in einer Wasserprobe gelösten Stoffe unspezifisch oxidiert werden, was mit der Abgabe von Elektronen an die Arbeitselektrode einhergeht. An einer weiteren Elektrode, der sogenannten Gegenelektrode, findet entsprechend ein Reduktionsprozeß statt, der Elektronen verbraucht. Der durch die wässrige Lösung fließende elektrische Strom wird erfaßt und bildet ein Maß für die je Zeiteinheit an den Elektroden umgesetzten Stoffmengen.

Die Arbeitsweise des erfindungsgemäßen Verfahrens ist neben dem Potential der Arbeitselektrode auch von der Wahl des Elektrodenmaterials und den Inhaltsstoffen der wässrigen Lösung abhängig.

Das Prinzip der Wasseranalytik mittels Elektrolyse ist an sich bekannt und an vielen Stellen im Einsatz. Es erlaubt - jeweils mit ganz spezifischen Elektrolyten und unter Verwendung an den Anwendungsfall angepaßter Elektrodenmaterialien - die Bestimmung zahlreicher Wasserinhaltsstoffe. Es ist sogar möglich, unter genau definierten Bedingungen (Elektrodenmaterial, Elektrolyt, Potential der Arbeitselektrode, etc.) bestimmte Elemente oder Verbindungen einzeln spezifisch nachzuweisen.

Die elektrochemische Bestimmung des CSB erfolgt hier hingegen mit einer unspezifischen Oxidation, so daß in einer Wasserprobe, in der sich eine Vielzahl der unterschiedlichsten Verbindungen befinden kann, die oxidierbaren Stoffe summarisch erfaßt werden. Das Material der Arbeitselektrode wird so gewählt, daß es die einzelnen Stoffe im elektrochemischen Prozeß möglichst nicht direkt oxidiert, sondern stattdessen hauptsächlich hochreaktive Verbindungen bildet, die ihrerseits die Wasserinhaltsstoffe unspezifisch angreifen und oxidieren. Ein solches Elektrodenmaterial ist Bleidioxid (PbO₂); Elektroden dieses Materials bilden in wässrigen Lösungen Ozon und OH-Radikale, die als ideale Oxidationsmittel für den gewünschten Zweck angesehen werden können.

Eine Bleidioxidelektrode dient zur unspezifischen elektrochemischen Oxidation von Wasserinhaltsstoffen als Verfahren zur einfachen meßtechnischen Erfassung des Summenparameters: chemischer Sauerstoffbedarf. Alle weiteren Randbedingungen (Arbeitselektrolyt, Material der Gegenelektrode, Art der Referenzelektrode, ja sogar das Potential der Arbeitselektrode) sind demgegenüber in weiten Bereichen variabel.

Die Arbeitselektrode arbeitet bei einem kontrollierten Potential. Da der Prozeß aber energie- und damit potentialabhängig ist, fußt der eine Pol der Spannungsquelle auf einer Referenzelektrode, die selbst nicht von Strom durchflossen wird und daher sehr potentialstabil ist.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Zeichnung näher dargestellt. Die Figuren zeigen Prinzipdarstellungen von Vorrichtungen die zur Durchführung des erfindungsgemäßen Verfahrens verwendet werden. Es zeigen:
Figur 1 eine Prinzipdarstellung eines Ausführungsbeispiels des erfindungsgemäßen Meßprinzips sowie
Figur 2 ein Ausführungsvariante mit Maßnahmen für einen kontinuierlichen Meßbetrieb.

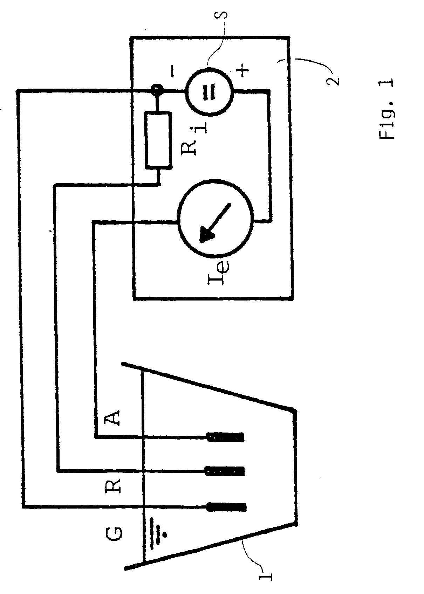

Bei der in der Figur 1 in Prinzipdarstellung wiedergegebenen Meßanordnung befinden sich in einem Meßgefäß 1, das von dem zu analysierenden Wasser kontinuierlich durchquert wird, drei Elektroden: eine Arbeitselektrode A, eine Gegenelektrode G und eine Referenzelektrode R. Die elektrische Beschaltung ist in einer separaten Einheit 2 vorgesehen, welche eine Spannungsquelle S enthält, die eine vorwählbare Festspannung abgibt. Ein Strommeßgerät Iₑ befindet sich in einer Serienschaltung zwischen der Gegen- und der Arbeitselektrode G bzw. A. Über einen Widerstand Rᵢ ist die Referenzelektrode zur Gegenelektrode G parallel geschaltet. Durch das eingestellte Spannungspotential werden an der Arbeitselektrode A die in der Wasserprobe gelösten Stoffe unspezifisch oxidiert, was durch Elektronenabgabe zu einem Stromfluß an dieser Elektrode führt. Die Oxidation erfolgt insbesondere indirekt. Bei einzelnen Stoffen kann es durchaus vorkommen, daß diese auch direkt oxidiert werden. Da aber die anderen Wasserinhaltsstoffe durch OH-Radikale oder Ozon ebenfalls oxidert werden, nehmen diese an der unspezifischen Oxidation teil. An der Gegenelektrode G findet ein entsprechender Reduktionsprozeß statt, wodurch Elektronen aufgenommen werden. Der durch die wässrige Lösung fließende elektrische Strom wird mittels des Strommeßgerätes erfaßt und bildet ein Maß für die je Zeiteinheit an den Elektroden umgesetzten Stoffmengen. Statt des Strommeßgerätes kann bevorzugt ein verfahrenstechnischer Steuer- oder Regelkreis nachgeschaltet sein, in dem in Abhängigkeit vom Stromwert Ausgangssignale erzeugt werden, welche ihrerseits Stellglieder wie Schieber oder dergleichen beeinflussen. Durch kontinuierliche Verstellung von Schiebern in Abhängigkeit vom Meßergebnis kann beispielsweise die Güte von Abwasser in einem Entsorgungsweg durch Steuerung der Mischung und/oder Einleitung in Abhängigkeit von der Wassergüte der Mischungskomponenten selbsttätig geregelt werden.

Die einzelnen zur Optimierung auch der Randparameter gewählten Materialen sollen nachfolgend kurz beschrieben werden:
- Als Arbeitselektrolyt wird 0,1-molare Kaliumsulfat- oder Natriumperchloratlösung verwendet; diese Elektrolyten sind wenig kostenaufwendig, verhalten sich inert und führen nicht zur Bildung schädlicher Stoffe, wie dies beispielsweise bei chloridhaltigen Elektrolyten (Chlorgas!) der Fall wäre. Als Material für die Gegenelektrode dient Platinblech. Hier kann, verbunden mit einer erheblichen Kostenersparnis, auch ein anderes, preiswerteres Material, vorzugsweise Titan, verwendet werden.
- Als Referenzelektrode dient ein Hg/HgSO₄-System; dies ist insbesondere im Zusammenhang mit den gewählten Elektrolyten günstig; die Wahl eines Systems, an dem Chlorid beteiligt ist, ist hingegen zu vermeiden.
- Das gewählte Arbeitspotential liegt zwischen 1 000 und 1 600 mV. Da die Elektrode unspezifisch arbeitet, funktioniert sie bei beliebigen Spannungen innerhalb dieses Bereichs. Im Anwendungsfall wird man ein festes Arbeitspotential einstellen.

Bei bestimmen Betriebsbedingungen kann es günstig sein, die Elektrode zu reinigen, wenn nämlich bestimmte Verbindungen in höheren Konzentrationen (beispielsweise Phenol in Konzentrationen über 100 mg/l) meßtechnisch störende Deckschichten auf der Bleidioxidelektrode bilden. Eine einfache Gegenmaßnahme besteht darin, daß die Elektrode im Anschluß an eine Messung in einem "sauberen" Elektrolyten mit hohem Potential arbeitet, so daß sie sich oxidativ reinigt. Ein derartiger Reinigungsvorgang kann in einem automatisiertem Prozeß periodisch selbsttätig durchgeführt werden. Bei dem dargestellten Ausführungsbeispiel ist dazu eine Vorrichtung zur Umsetzung der Elektroden in ein sauberes Medium (bzw. eine entsprechende Änderung der Zufuhr des Meßmediums) vorzusehen, wobei periodisch mit dem Umsetzen der Elektroden der Meßvorgang unterbrochen und das Potential erhöht wird. Danach kann die Messung mit den gereinigten Elektroden fortgesetzt werden.

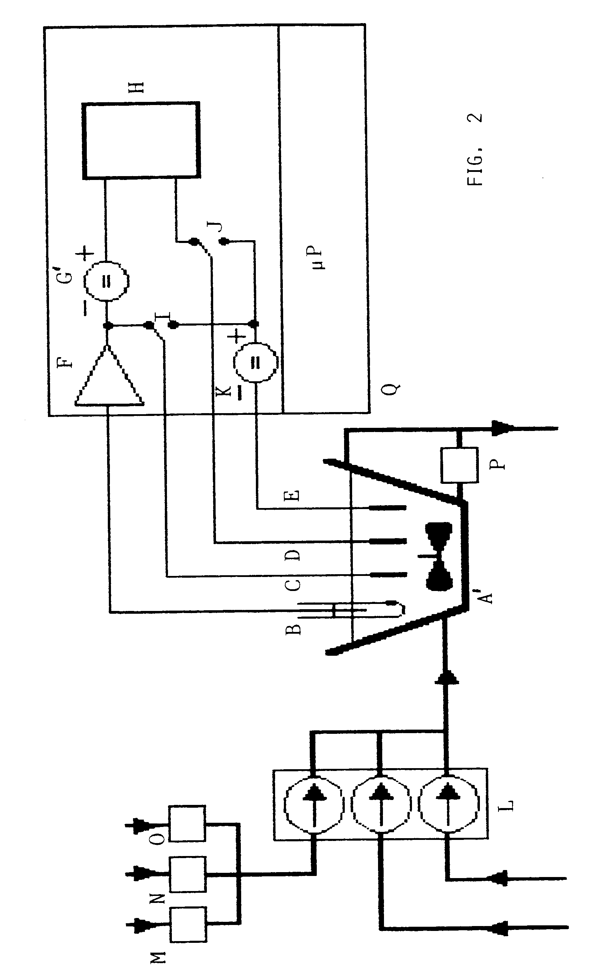

Bei der in Figur 2 wiedergegebenen Ausführungsvariante sind die Elemente A bis K für die Untersuchung einzelner Proben erforderlich (manuelle Methode, Einzelbestimmung)
Ein Reaktionsgefäß Aʹ ist mit Überlauf und Rührer versehen und enthält die Probenflüssigkeit. Eine Referenzelektrode B bildet eine Bezugselektrode zweiter Art mit Diaphragma, die vorzugsweise als Hg/HgSO₄-System ausgeführt ist. Eine Gegenelektrode C besteht vorzugsweise aus Platinblech - andere Metalle sind als Material möglich. Bei einer Arbeitselektrode D ist Bleidioxid auf einem metallischem Träger abgeschieden.

Zur Regeneration der vorstehend beschriebenen Anordnung ist eine Regenerationselektrode E zur Benutzung zur Wiederherstellung der Eigenschaften der Elektroden C und D vorgesehen.

Ein Impedanzwandler F weist einen extrem hochohmigem Eingang auf und hält die Elektrode B stromfrei. Eine Konstantspannungsquelle Gʹ dient zur Erzeugung des Potentials der Arbeitselektrode in Verbindung mit der potentialstabilen Referenzelektrode B. Eine Meßwertauswert- und Anzeigevorrichtung H dient zur Messung des Stroms durch die Arbeitselektrode und Umrechnung des elektrischen Stroms in die Größe: "mg/l CSB". Zusätzlich erfolgt eine Anzeige des Stromwerts oder des CSB direkt.

Ein Umschalter I dient zur Schaltung der Gegenelektrode in den Regenerationsstromkreis, während ein weiterer Umschalter J dieselbe Aufgabe im Hinblick auf die Arbeitselektrode D hat. Bei der Regeneration wird eine Konstantspannungsquelle K als Energiequelle eingeschaltet.

Die nachfolgend beschriebenen Baugruppen dienen insbesondere für eine automatisierten und kontinuierliche Bestimmung von CSB:
Eine Dreikanalpumpe L dient zur Dosierung von Probenwasser bzw. von zwei Kalibrierlösungen mit bekanntem CSB (oberer Pumpenkanal), sowie zur Zugabe von Elektrolytkonzentrat (mittlerer Pumpenkanal) und gegebenenfalls Verdünnungswasser (unterer Pumpenkanal) für Proben mit sehr hohem CSB.

Ein Magnetventi M leitet im Aktivierungsfall der Pumpe Probenwasser zu, ein Magnetventil N leitet im Aktivierungsfall der Pumpe Kalibrierlösung für den unteren Kalibrierpunkt zu und ein Magnetventil O leitet im Aktivierungsfall der Pumpe Kalibrierlösung für den oberen Kalibrierpunkt zu. Ein Magnetventil P dient zur Entleerung des Reaktionsgefäßes. Ein elektronischer Mikroprozessor µP dient zur Steuerung des Ablaufprogramms bei automatischer Messung, wobei die elektrischen Steuerleitungen zu den Elementen L bis P nicht dargestellt sind.

Durch das Ablaufprogramm gesteuert wird kontinuierlich Probenflüssigkeit in das Reaktionsgefäß gefördert und mit Elektrolytkonzentrat und gegebenenfalls Verdünnungswasser versetzt. Dies gilt insbesondere für Proben mit hohem CSB, wobei der Verdünnungsfaktor bei der Berechnung des Meßwerts durch den Mikroprozessor automatisch berücksichtigt wird.

Das Ablaufprogramm nimmt auch automatisch die Kalibrierung der Meßanordnung mit zwei Kalibrierlösungen und die Regeneration der Arbeits- und der Gegenelektrode vor. Hierfür wird das Reaktionsgefäß über das Magnetventil P entleert und nacheinander mit Kalibrierlösungen befüllt. Anschließend wird die automatische und kontinuierliche Messung fortgesetzt.

Die Erfindung beschränkt sich in ihrer Ausführung nicht auf das vorstehend angegebene bevorzugte Ausführungsbeispiel.

## Patentansprüche

1. Verfahren zur Bestimmung des chemischen Sauerstoffbedarfs von Wasser,
**dadurch gekennzeichnet,**
daß die Bestimmung durch unspezifische elektrochemische Oxidation der in einer Wasserprobe gelösten Stoffe bei kontrolliertem Potential erfolgt, wobei der über eine Bleidioxid-Arbeitselektrode durch die wässrige Lösung fließende Strom ein Maß für die je Zeiteinheit an den Elektroden umgesetzten Stoffmengen bildet.

2. Verfahren nach Anspruch 1, **dadurch gekennzeichnet,** daß das kontrollierte Potential derart gewählt ist, daß die einzelnen Stoffe im elektrochemischen Prozeß insbesondere nicht direkt oxidiert, sondern stattdessen hochreaktive Verbindungen gebildet werden, die ihrerseits die Wasserinhaltsstoffe unspezifisch angreifen und oxidieren und insbesondere zwischen 1 000 und 1 600 mV beträgt.

3. Verfahren nach einem der vorangehenden Ansprüche, **dadurch gekennzeichnet,** daß das Elektrodenmaterial derart gewählt ist, daß es in wässrigen Lösungen Ozon und/oder OH-Radikale bildet.

4. Verfahren nach einem der vorangehenden Ansprüche, **dadurch gekennzeichnet,** daß als Arbeitselektrolyt insbesondere 0,1-molare Kalium- oder Natriumsulfat- oder Natriumperchloratlösung benutzt wird.

5. Verfahren nach einem der vorangehenden Ansprüche, **dadurch gekennzeichnet,** daß als Material für die Gegenelektrode Platinblech oder Titan verwendet wird.

6. Verfahren nach einem der vorangehenden Ansprüche, **dadurch gekennzeichnet,** daß als Referenzelektrode ein Hg/HgSO₄-System bzw. ein System an dem keine Chlorid beteiligt ist, dient.

7. Verfahren nach einem der vorangehenden Ansprüche, **gekennzeichnet durch** die intermittierende oxidative Reinigung der Elektrode in einem unverschmutzten Elektrolyten.

8. Verfahren nach einem der vorangehenden Ansprüche, **dadurch gekennzeichnet,** daß der Strom eine Stellgröße in einem elektrochemisch geregelten System bildet und die Stellgröße insbesondere zur Steuerung von Schiebern in einem Wasserver- oder -entsorgungssystem dient.

9. Verwendung einer Vorrichtung zur Durchführung des Verfahrens zur Bestimmung des chemischen Sauerstoffbedarfs von Wasser nach einem der vorangehenden Ansprüche, wobei die Vorrichtung folgende Geräte und Einrichtungen aufweist:
a) eine eine vorwählbare Festspannung abgebende Spannungsquelle,
b) eine bei kontrolliertem Potential arbeitende Bleidioxid-Arbeitselektrode an der die in der Wasserprobe gelösten Stoffe unspezifisch oxidieren,
c) eine stromfrei gehaltene Referenzelektrode und eine Gegenelektrode, durch die Strom fließt, wobei die Referenzelektrode über einen Widerstand zur Gegenelektrode parallel geschaltet ist, sowie
d) ein in Serienschaltung zwischen der Gegen- und der Arbeitselektrode angeordnetes Strommeßgerät, welches den durch die Wasserprobe fließenden elektrischen Strom erfaßt.

## Claims

1. Method of determining the chemical oxygen demand of water, characterised in that it is determined by non-specific electrochemical oxidation of the substances dissolved in a sample of water at a controlled potential, wherein the current flowing through a lead dioxide working electrode and through the aqueous solution constitutes a measurement of the quantities of substance reacted at the electrodes per unit of time.

2. Method according to claim 1, characterised in that the controlled potential is selected so that the individual substances are not oxidised directly in the electrochemical process but instead highly reactive compounds are formed which in turn non-specifically attack and oxidise the substances contained in the water, in particular between 1,000 and 1,600 mV.

3. Method according to one of the preceding claims, characterised in that the electrode material is selected so as to form ozone and/or OH radicals in aqueous solutions.

4. Method according to one of the preceding claims, characterised in that, in particular, 0.1 molar potassium or sodium sulphate or sodium perchlorate solution is used as the working electrolyte.

5. Method according to one of the preceding claims, characterised in that sheet platinum or titanium is used as the material for the counterelectrode.

6. Method according to one of the preceding claims, characterised in that an Hg/HgSO₄ system or a system in which no chloride is involved is used as the reference electrode.

7. Method according to one of the preceding claims, characterised by the intermediate oxidative cleaning of the electrode in an uncontaminated electrolyte.

8. Method according to one of the preceding claims, characterised in that the current forms a regulated quantity in an electrochemically regulated system and the regulated quantity is used, in particular, to control slide valves in a water supply or removal system.

9. Use of an apparatus for carrying out the method for determining the chemical oxygen demand of water according to one of the preceding claims, wherein the apparatus comprises the following equipment and devices:
a) a voltage source which delivers a preselectable fixed voltage,
b) a lead dioxide working electrode operating under controlled potential, at which the substances dissolved in the water sample oxidise non-specifically,
c) a reference electrode maintained free from current and a counterelectrode through which current flows, the reference electrode being connected in parallel to the counterelectrode via a resistor, and
d) a current measuring device connected in series between the counterelectrode and the working electrode, this current measuring device determining the electric current flowing through the water sample.

## Revendications

1. Procédé de détermination de la demande chimique en oxygène de l'eau, caractérisé en ce que la détermination est accomplie à potentiel contrôlé par oxydation électrochimique non spécifique des substances dissoutes dans un échantillon d'eau, le courant qui circule dans la solution aqueuse par l'intermédiaire d'une électrode de travail en dioxyde de plomb constituant une mesure des quantités de substances converties aux électrodes par unité de temps.

2. Procédé selon la revendication 1, caractérisé en ce que le potentiel contrôlé est choisi de telle manière que les différentes substances ne soient pas oxydées en particulier directement dans le processus électrochimique mais qu'il se forme des composés hautement réactifs qui pour leur part attaquent et oxydent de manière non spécifique les substances contenues dans l'eau et qu'il soit compris en particulier entre 1 000 et 1 600 mV.

3. Procédé selon l'une des revendications précédentes, caractérisé en ce que le matériau des électrodes est choisi de telle manière qu'il forme dans les solutions aqueuses de l'ozone et/ou des radicaux OH.

4. Procédé selon l'une des revendications précédentes, caractérisé en ce que l'on utilise comme électrolyte de travail en particulier une solution 0,1 fois molaire de sulfate de potassium ou de sodium ou de perchlorate de sodium.

5. Procédé selon l'une des revendications précédentes, caractérisé en ce que l'on utilise comme matériau pour la contre-électrode une lame de platine ou le titane.

6. Procédé selon l'une des revendications précédentes, caractérisé en ce que l'on utilise comme électrode de référence un système Hg/HgSO₄ ou un système auquel ne participe aucun chlorure.

7. Procédé selon l'une des revendications précédentes, caractérisé par la purification oxydative intermittente des électrodes dans un électrolyte non pollué.

8. Procédé selon l'une des revendications précédentes, caractérisé en ce que le courant constitue une variable de réglage dans un système à régulation électrochimique et la variable de réglage sert en particulier pour la commande de vannes dans un système d'alimentation en eau ou d'évacuation de l'eau.

9. Utilisation d'un dispositif pour la mise en oeuvre du procédé de détermination de la demande chimique en oxygène de l'eau selon l'une des revendications précédentes dans laquelle le dispositif comporte les appareils et installations suivants :
a) une source de tension qui délivre une tension continue qui peut être choisie au préalable,
b) une électrode de travail en dioxyde de plomb qui fonctionne à potentiel contrôlé et au niveau de laquelle les substances dissoutes dans l'échantillon d'eau s'oxydent de manière non spécifique,
c) une électrode de référence maintenue sans courant et une contre-électrode qui est traversée par un courant, l'électrode de référence étant montée en parallèle avec la contre-électrode par l'intermédiaire d'une résistance, et
d) un ampèremètre disposé en série entre la contre-électrode et l'électrode de travail et qui mesure le courant électrique qui traverse l'échantillon d'eau.
